# EUROPEAN PATENT APPLICATION

(11) **EP 2 796 859 A1**
(43) Date of publication of application: **29.10.2014**
(21) Application number: 13165451.9
(22) Date of filing: 25.04.2013
(51) Int. Cl.: G01N 21/64, G01N 33/58

(54) **Quantification of a functionalized surface**

(71) Applicant: Biocartis SA, 1015 Lausanne (CH)
(72) Inventor: She, King Shing Joseph, 1009 Pully (CH)
(74) Representative: Foy, Nicolas

(57) **Abstract**

The present invention relates to a method for quantifying at a functional group (1) functionalizing a surface (2). First, the method comprising the step of providing at a surface (2) having micrometric dimensions functionalized with at a functional group (1). The method includes a step of contacting the surface (2) with a solution: Said solution comprises at a first reagent (4) at a first concentration and a second reagent (5) at a second concentration, the ratio between the first concentration and the second concentration being predetermined. Said first reagent (4) and second reagent (5) are designed for selectively coupling to a functional group (2) with respective affinities, the ratio of said affinities being predetermined. The first reagent (4) is labelled for emitting fluorescence and the second reagent (5) is un-labelled. Then, the fluorescence of said first reagent (4) on the surface to quantify said functional group (1) therefrom.

## Description

The present invention relates to a method for quantifying at least a functional group functionalizing a surface.

Functionalized surfaces are important tools for bio-sensing technologies. Therefore, methods to assess the functionalization of the surface are needed to guarantee the reliability of those technologies and quality control methods are useful to provide technologies with proven efficiency and accuracy.

Different functional groups have been described as efficient entities to attach biological materials to a surface. Such materials could be grafted to a surface presenting for example either an amine (-NH₂), a carboxylic acid (-COOH) or derivatives of thereof or a hydroxyl group (-OH). Those chemical functional groups enable the covalent attachment of a wide range of molecules onto the surface using well-established coupling protocols from literatures.

From manufacturing's point of view, the detection and characterisation of certain functional groups, such as surface -COOH groups, on functionalized surfaces are not well established and many usual characterisation techniques present several drawbacks for both an accurate and a routine utilization. Indeed, firstly those methods, such as infra-red spectroscopy and X-ray photoelectron spectroscopy (XPS), are expensive to operate and secondly, they often present low specificity, as being designed for targeting a charged surface rather than a specific functional group. Thus, concerning the carboxylic acid grafted surface, those methods often failed to distinguish carboxylic acid from other oxygen-containing functional groups. Moreover, the methods mentioned above are usually not suitable for providing information when it comes to surfaces with small dimensions.

There are other emerging techniques. One such method relies on the adsorption/desorption of a colormetrically active or fluorescent molecule onto a charged surface. For example, one method describes the adsorption of positively charged toluidine blue O on a negatively charged surface, as mentioned in the article from Wang, Y. (Surface graft polymerization of SU-8 for bio-MEMS applications, Journal of Micromechanics and Microengineering. 2007, Vol.°17, pages 1371 to 1380). In general, such methods either consist of quantifying the remaining molecules in the bulk solution after adsorption to the surface, or quantifying the eluted molecules from the surface after desorption. Although such methods can be applied to any charged surface (low specificity), the quantification step cannot precisely distinguish specific from non-specific binding of the colorimetrically active or fluorescent molecule. For micro-meter range or smaller surfaces, the percentage of depletion is too small to be assessed accurately.

The method called Fluorescence Labelling of Surface Species (FLOSS) is an attractive alternative for surface functional group characterisation, as mentioned in the article regarding the -COOH group, by Pellenbarg, T.( Detecting and quantifying oxygen functional groups on graphite nanofibers by fluorescence labelling of surface species, Carbon. 2010, Vol.°48, pages 4256 to 4267). In this method, fluorescent molecules that specifically react to -COOH group are used to label the surface by covalent bond formation. The labelled surface is then characterised by fluorescence spectroscopy. Although the surface functional group density can be directly measured from the surface with the FLOSS methodology, it is only useful for relatively large surfaces (in the range of mm). For micro-meter range or smaller surfaces, we observed that the method is not quantitative, and that for a given surface, a measured fluorescence may correspond to two functional group concentrations. Consequently, the FLOSS methodology is not applicable to micro-meter range or smaller surfaces. There is, so far, no proposed solution to overcome those issues when it comes to characterizing micro-meter range or smaller surfaces.

The present invention aims to remedy all or part of the disadvantages mentioned above.

The present invention fulfils these objectives by providing a method for quantifying at least a functional group functionalizing a surface, the method comprising the successive steps of:
a. Providing at least a surface having micrometric dimensions functionalized with at least a functional group,
b. Contacting the surface with a solution,
   i. said solution comprising at least a first reagent at a first concentration and a second reagent at a second concentration, the ratio between the first concentration and the second concentration being predetermined;
   ii. said first reagent and second reagent being designed for selectively coupling to one of the at least a functional group with respective affinities, the ratio of said affinities being predetermined;
   iii. the first reagent being labelled for emitting a fluorescence and the second reagent being un-labelled;
c. Measuring the fluorescence of said first reagent coupled to the surface and quantifying said functional group therefrom.

Thus, the present invention solves the problem by contacting the functionalized surface with a solution comprising a first reagent being labelled at a first concentration and second reagent being un-labelled at a second concentration by avoiding the self-quenching issue observed with usual methods. Indeed, when contacting the functionalized surface with the solution, the first reagent and the second reagent react with the functional groups grafted on a surface and the coupling reaction is driven by the respective affinities of the first reagent and of second reagent with the functional group. Besides, the first reagent further comprises a fluorescent molecule designed for emitting fluorescence. Thus, said functional group of the surface is coupled either with the first reagent or with the second reagent, so that each functional group will be coupled either with the first reagent or with the second unreagent. Hence, the method according to the present invention physically spaces out the first reagent on the target surface therefore avoiding the self-quenching phenomena.

The method according to the present invention also permits to quantify the functional group on the surface. Indeed firstly, the concentration of the first reagent and the concentration of the second reagent are predetermined parameters. Secondly, the respective affinity ratio mentioned above provides information about the affinity of the first reagent and of the second reagent with the functional group. Hence, after measuring the fluorescence of the surface, the presence of the targeted functional group can be quantified by taking into account the ratio of the concentrations and the respective affinities ratio between the first reagent and the second reagent with the functionalized group.

The method according to the present invention also permits the quantification of different functional groups grafted on a surface. For example, the method according to the present invention also permits the independent quantification of all functional groups on a surface functionalized with hydroxyl (-OH) and amine (-NH₂) groups.

When the surface comprises different functional groups, for each functional group a couple of first reagents and second reagents will be chosen in order to target specifically said functional group. The first reagent and the second reagent will be at a predetermined concentration ratio and will present respective predetermined affinities with said functional group.

According to an embodiment, the method further comprises, prior to step a), an activation step to activate the functional group of the surface. This embodiment aims at preparing the functional group designed for being coupled with the first reagent and with the second reagent. Thus, the reactivity of the functional group grafted on the surface can be triggered by this activation step. After this activation step, an inactive functional group toward the first reagent and/or the second reagent can be turned into a reactive functional group ready for being coupled with the first reagent and/or with the second reagent.

In an embodiment, said activation proceeds via at least a chemical reaction. Thus, this chemical reaction permits to promote or to trigger the coupling of the first reagent and of the second reagent with the functional group. Such a chemical activation is simple to implement.

According to an embodiment, the ratio between the first concentration and the second concentration is between about 1.99 to about 50.50, preferably between about 2:98 to about 25:75, more preferably between about 3:97 to about 10:90, and more preferably between about 4:96 to about 7:93.

According to an embodiment, the surface is comprised on a microparticle. A large number of microparticles can be measured simultaneously to provide information regarding functional group density on a single microparticle. Moreover, the methods enable having details information regarding one specific population or between different populations with different codes in one single measurement.

In an embodiment, the first reagent and/or the second reagent are chosen among proteins, antibodies, peptides, nucleic acids or a mixture of thereof.

According to an embodiment, the functional group is a carbonyl group chosen among carboxylic acids or derivative thereof, ketones, aldehydes. Thus, in this embodiment the first reagent and the second reagent can be coupled to the functional group by using any group designed for being coupled with at least a functional group according to this embodiment.

In an embodiment, the first reagent and/or the second reagent are coupled to the functional group of the surface via a peptide bond.

According to an embodiment, the first reagent and/or the second reagent present an amine group involved in the coupling with the functional group of the surface. Thus, according to this embodiment the functional group can be chosen among any functional group design for being coupled to an amine group.

In an embodiment, the surface is made of glass, silicon, or polymer.

According to an embodiment, the first reagent is obtained by labelling the second reagent. In many fluorescence-based characterisation methods, a specific fluorescent label is developed for the detection, whereas, in the method according to the present invention, any fluorescent molecule that can be conjugated to the second reagent can be used.

In an embodiment, the second reagent is streptavidin.

According to an embodiment, the first reagent is labelled with FITC (CAS Number 3326-32-7), rhodamine (CAS Number 81-88-9), cyanine 3 (CAS Number 605-91-4), cyanine 5 (CAS Number 14187-31-6), AlexaFluor® 488, Alexa Fluor® 532, Alexa Fluor® 647 (Alexa Fluor® dyes commercialized by Molecular Probes, Switzerland), ATTO 488, ATTO 532, ATTO 647N (ATTO dyes commercialized by ATTO-TEC GmbH, Germany) or mixture of thereof.

In an embodiment, the streptavidin is labelled with ATTO 647N.

The present invention is further illustrated by the following detailed description set forth in view of the appended drawing, which represent an exemplary and explanatory embodiment of a method for quantifying at least a functional group functionalizing a surface

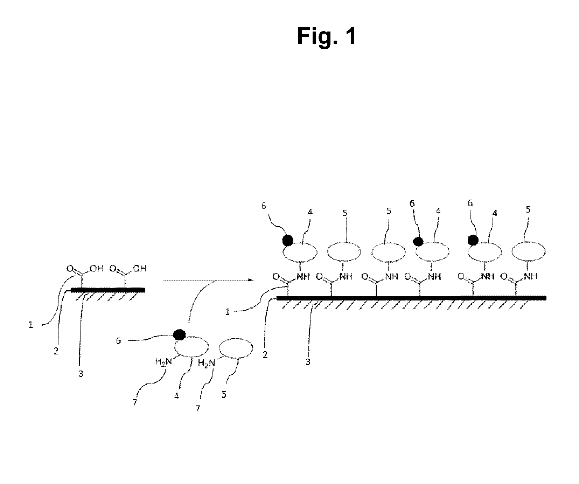

Figure 1 illustrates a method according to the invention for quantifying a surface functionalized with carboxylic acid (COOH).

In a method, according to the present invention, for quantifying at least a functional group (1) functionalizing a surface (2), said surface (2) is comprised on a microparticle (3) and is functionalized with carboxylic acids groups (-COOH) as shown on figure 1.

To quantify the functional group (1) that is carboxylic acid group, the method is initiated with an activation step consisting in contacting the surface (2) with an activating solution comprising a peptide coupling agent, such as EDC (N-(3-Dimethylaminopropyl)-N'-ethylcarbodiimide hydrochloride).HCI (5 mg) and sulfo-NHS (12.5 mg - N-Hydroxysulfosuccinimide sodium salt) in 3.6 mL of activation buffer (100 mM MES, 0.3% (v/v) Tween®-20, pH 2.3). In the activation step, the microparticle (3) is immersed in the activation solution for about 60 minutes at room temperature.

After the activation step, the microparticle (3) is rinsed to remove any excess of chemicals with a washing buffer (10 mM phosphate, 2.7 mM potassium chloride, 137 mM sodium chloride, 0.3% (v/v) Tween®-20, pH 7.4).

Subsequently, the surface (2) is contacted with a solution that comprises a first reagent (4) and a second reagent (5). The second reagent (5) is a streptavidin and the first reagent (4) is a labelled streptavidin, carrying as a at least a fluorophore (6) ATTO 647 N commercialized by ATTO-TEC GmbH, Germany. The fluorophore (6) ATTO 647 N is attached to the first reagent (4) via at least a peptide bond implying at least a lysine residue. An amine group (7) from the streptavidin is designed for being coupled to the carboxylic group of the surface (2) via a peptide bond. In the solution, the first reagent (4) is present at a first concentration C1 and the second reagent (5) is present at a second concentration C2, the ratio C1/C2 between the first concentration C1 and the second concentration C2 is predetermined. Practically, the solution containing 60 µL of 5% ATT0647N-streptavidin and 95% streptavidin and Phosphate buffered saline (PBS) at 500 µg/mL is added to the microparticle (3) in 2.1 mL of coupling buffer (10 mM sodium acetate, 0.3% (v/v) Tween®-20 (commercialized by Sigma-Aldrich, CAS # 9005-64-5), pH 4.8) and the reaction is run at room temperature for 30 minutes. The microparticle (3) is then washed with the washing buffer to remove any excess of reagents.

To quantify the presence of functional group (1) on the surface (2), the microparticle (3) is first placed under a fluorescence microscope for quantifying measurement. Subsequently, the presence of carboxylic groups on the surface (2) is quantified by taking into account the measured fluorescence with respect to the concentration ratio of C1/C2, between the first concentration C1 and the second concentration C2.

Other embodiments of the invention will be apparent to those skilled in the art from consideration of the specification and practice of the invention disclosed herein. It is intended that the specification and examples be considered as exemplary only, with the true scope and spirit of the invention being indicated by the following claims.

## Claims

1. A method for quantifying at least a functional group (1) functionalizing a surface (2), the method comprising the successive steps of:
a. Providing at least a surface (2) having micrometric dimensions functionalized with at least a functional group (1),
b. Contacting the surface (2) with a solution,
i. said solution comprising at least a first reagent (4) at a first concentration and a second reagent (5) at a second concentration, the ratio between the first concentration and the second concentration being predetermined;
ii. said first reagent (4) and second reagent (5) being designed for selectively coupling to one of the at least a functional group (1) with respective affinities, the ratio of said affinities being predetermined;
iii. the first reagent (4) being labelled for emitting a fluorescence and the second reagent (5) being un-labelled;
c. Measuring the fluorescence of said first reagent (4) coupled to the surface (2) and quantifying said functional group (1) therefrom.

2. A method according to claim 1, wherein said method further comprises, prior to step a), an activation step to activate the functional group (1) of the surface (2).

3. A method according to claim 2, wherein said activation proceed via at least a chemical reaction.

4. A method according to any one of claims 1 to 3, wherein the ratio between the first concentration and the second concentration is between about 1.99 to about 50.50, preferably between about 2:98 to about 25:75, more preferably between about 3:97 to about 10:90, and more preferably between about 4:96 to about 7:93.

5. A method according to any one of claims 1 to 4, wherein the surface (2) is comprised on a microparticle (3).

6. A method according to any one of claims 1 to 5, wherein the first reagent (4) and/or the second reagent (5) are chosen among proteins, antibodies, peptides, nucleic acids or a mixture of thereof.

7. A method according to any one of claims 1 to 6, wherein the functional group (1) is a carbonyl group chosen among carboxylic acids or derivative thereof, ketones, aldehydes.

8. A method according to any one of claims 1 to 7, wherein the first reagent (4) and/or the second reagent (5) are coupled to the functional group (1) of the surface (2) via a peptide bond.

9. A method according to any one of claims 1 to 8, wherein the first reagent (4) and/or the second reagent (5) present an amine group involved in the coupling with the functional group (1) of the surface (2).

10. A method according to any one of claims 1 to 9, wherein the surface (2) is made of glass, silicon, or polymer.

11. A method according to any one of claims 1 to 10, wherein the first reagent (4) is obtained by labelling the second reagent (5).

12. A method according to claim 11, wherein the second reagent (5) is streptavidin.

13. A method according to any one of claims 1 to 12, wherein the first reagent (4) is labelled with FITC, rhodamine, cyanine 3, cyanine 5, AlexaFluor® 488, Alexa Fluor® 532, Alexa Fluor® 647, ATTO 488, ATTO 532, ATTO 647N or mixture of thereof.

14. A method according to claim 12 in combination with 13, where the streptavidin is labelled with ATTO 647N.
